# EUROPEAN PATENT APPLICATION

(11) **EP 3 812 757 A1**
(43) Date of publication of application: **28.04.2021**
(21) Application number: 19204681.1
(22) Date of filing: 22.10.2019
(51) Int. Cl.: G01N 29/024, G01N 29/032, G01N 29/48

(54) **METHOD AND APPARATUS FOR DETECTING A PROPERTY OF A LIQUID MEDIUM, UREA SENSOR SYSTEM, COMPUTER PROGRAM PRODUCT AND COMPUTER-READABLE STORAGE MEDIUM**

(71) Applicant: TE Connectivity Norge AS, 5258 Blomsterdalen (NO)
(72) Inventor: BOE, Hakon, 5081 Bergen (NO); LIE, Bjornar, 5081 Bergen (NO)
(74) Representative: Grünecker Patent- und Rechtsanwälte PartG mbB

(57) **Abstract**

The invention relates to a method and apparatus (1) for detecting a property of a liquid medium (9), in particular for detecting a property representative of disturbances (11) in the liquid medium (9), to an urea sensor system, a computer program product and a computer-readable storage medium. In methods and apparatuses (1) of the art commonly, the signal to noise ratio (SNR) is determined. The SNR is, however, inaccurate and/or not suitable to determine disturbances (11) in the liquid medium (9) due to drift of a pulse generator (3) and/or of a receiver because of a temperature change. This problem is overcome by the inventive method by comprising the steps: generating an ultrasonic pulse (21); receiving at least a first echo (23) and a second echo (29) of the ultrasonic pulse (21) transmitted through the liquid medium (9); generating an amplitude signal (27, 31) for each of the at least two echoes (23, 29); calculating an amplitude signal ratio between the amplitude signal (27) of the first echo (23) and the amplitude signal (31) of the second echo (29); and determining at least one property signal representing the property of the liquid medium (9).

## Description

The invention relates to a method and an apparatus for detecting a property of a liquid medium, in particular for detecting a property representative of disturbances in the liquid medium, to a urea sensor system, a computer program product and a computer-readable storage medium.

In methods and apparatuses for detecting a property of a liquid medium of the art commonly, the signal to noise ratio (SNR) is determined. The SNR is, however inaccurate and/or not suitable to accurately determine disturbances in the liquid medium. One problem of the art is a drift of a pulse generator and/or of a receiver, which may for instance occur because of a temperature change.

An object of the present invention is therefore to provide a method, an apparatus and the corresponding computer program product or computer readable storage medium, which cancel out properties of a measurement apparatus and which provides more accurate measurements in the disturbed liquid media.

The method of the present invention solves the above problem in that it comprises the following steps: generating an ultrasonic pulse; receiving at least a first echo and a second echo of the ultrasonic pulse transmitted through the liquid medium; generating an amplitude signal for each of the at least two echoes; calculating an amplitude signal ratio between the amplitude signal of the first echo and the amplitude signal of the second echo; and determining at least one property signal representing the property of the liquid medium.

Accordingly, the apparatus of the present invention solves the above problems by comprising a pulse generator configured to generate an ultrasonic pulse; a receiver, configured to receive at least a first echo and a second echo of the ultrasonic pulse transmitted through the liquid medium; and a calculator unit configured to find and calculate an amplitude signal for each of the at least two echoes, to calculate an amplitude signal ratio between the amplitude signal of the first echo and the amplitude signal of the second echo and to determine at least one property signal representing the property of the liquid medium.

The inventive urea sensor system for determining at least one property of an urea solution, in particular for detecting a property representative of disturbances in the urea solution in that the urea sensor system comprises an inventive apparatus.

The present invention solves the above problems for the computer program product and the computer-readable storage medium by comprising instructions which, when executed by a computer, cause the computer to carry out the steps of the inventive method.

The method, apparatus, sensor system, computer program product and computer readable storage medium may be further improved by additional technical features. Those additional technical features may be arbitrarily combined with each other or omitted if the technical effect obtained by the omitted technical feature is not essential to the present invention.

The embodiment of the present disclosure may in particular the applied in the field of urea-containing liquid media.

When sending an ultrasonic pulse through a liquid medium, for example through an aqueous solution, bubbles, in particular gas bubbles and other disturbances will affect the pulse. A signal may be disturbed to such an extent that further measurements based on transmitting and receiving a reflected ultrasonic pulse may not be performed accurately any longer. Within this disclosure, the features liquid medium and aqueous solution may be replaced by each other. Thus, an embodiment denoting an aqueous solution may be applied to a more general liquid medium as well.

According to one aspect of the inventive apparatus, the pulse generator and the receiver may be combined in a transducer, which allows generating an ultrasonic pulse and receiving echoes, preferably at different times.

As mentioned above, measuring apparatuses determining the signal to noise ratio, as applied in the art, are disadvantageous as the signal-to-noise ratio may be different for different ultrasonic transducers and varies depending on the temperature, the life cycle of the transducer or due to further parameters.

The embodiment according to this disclosure provide an innovative way to determine a property of the liquid medium like an aqueous solution, in particular to measure the disturbance in the aqueous solution and preferably detecting said disturbance in an urea solution.

After generation of the ultrasonic pulse, said pulse may be transmitted through a sample volume containing the liquid medium. There the pulse impacts on a reflector reflecting back the pulse towards the transducer that generated the pulse.

At the transducer, the ultrasonic pulse is reflected again and performs a second round trip in the sample volume. Thus, a multitude of echos impacts on the transducer and is transformed into an electric signal. This electrical signal representative of the amplitude of the corresponding echo may be used to calculate an amplitude signal for each of the at least two echoes. This calculation is preferably performed by a calculating unit, which may be embodied as a field programmable gate area, a microcontroller or any suitable integrated circuit.

The same or an additional calculating unit may perform the calculation of the amplitude signal ratio between the amplitude signal of the first echo and the second echo.

Finally, at least one property signal is determined, wherein said property signal represents the property of the liquid medium. In particular, the property signal may represent the presence of any disturbance in the liquid medium.

As mentioned above, the embodiment of the present disclosure may particularly be applied in the field of urea solutions applied in diesel engines for cleaning diesel exhaust fumes. Commonly, speed of sound measurements may be applied for detecting the type and/or quality of the liquid medium, whereas in particular applying urea, said aqueous solution tends to produce bubbles, which renders measurements of the speed of sound inaccurate. It is therefore advantageous to precisely and accurately determine whether bubbles are present in the urea solution and to which extend. It is further advantageous to additionally cancel out the influence of present bubbles when determining the speed of sound in the liquid medium.

The present invention has the advantage that properties of the ultrasonic transducer are canceled out, i.e. do not influence the measurement. This may e.g. be advantageous in case of temperature drift, which would otherwise alter the properties of the transducer.

If according to the invention, the presence of disturbances, e.g. in the form of bubbles, is detected, possible further steps may be performed depending on the property signal.

Another embodiment of the method comprises the step of determining a time of flight for the first echo for calculating a measured speed of sound and providing a measured speed of sound signal representing the measured speed of sound. The speed of sound may be calculated by means of the time of flight and a known distance the ultrasonic pulse traveled.

In the corresponding apparatus, the calculating unit may provide a calculator that divides the distance traveled by the time of flight of the first echo.

The method may be further improved by comprising the steps of determining a compensation factor based on the amplitude signal ratio and a predetermined calibration ratio; determining a calculated speed of sound in the liquid medium at least based on the measured speed of sound and the compensation factor; and providing a calculated speed of sound signal representing the calculated speed of sound. this has the advantage that a speed of sound of the ultrasound pulse in a liquid medium without any disturbance may be determined.

The predetermined calibration signal ratio may be determined during calibration and/or provided in a storage medium. Thus, the predetermined calibration signal ratio may be determined prior to the ultrasonic measurement or provided in and read from a storage location, where it has been stored prior to the measurement.

The calculated speed of sound may further depend on an empirically found variable.

According to another embodiment, the method further comprises the step of comparing the amplitude ratio signal with a predetermined amplitude ratio threshold, wherein if the amplitude ratio signal exceeds the amplitude ratio threshold, the method is re-initiated and/or an alert signal is provided.

This embodiment has the advantage that it may be accurately determined whether the property of the liquid medium may be reliably measured or not, e.g. because of too much disturbances, like too much bubbles present in the liquid medium, which would hinder an accurate measurement. In particular in case of the urea solution, where urea tends to produce bubbles caused by vibrations, the extent of the number of bubbles generated in the liquid medium may constitute a critical parameter and needs to be monitored. This embodiment allows indicating such an excessive presence of bubbles or more general: the excessive presence of disturbances or even more general of the property of the liquid medium exceeding a predetermined threshold. One object of this embodiment is to indicate the exceeded threshold, wherein the outcome of this comparison may trigger further method steps, as e.g. the re-initialization or restart of the method. It is also conceivable that a detected exceeded threshold may initiate a further method step in which the number of bubbles in the liquid medium, in particular a urea solution, is reduced.

The embodiment of the apparatus according to this method may comprise a comparator module or unit, which may be implemented in the calculating unit or may be provided separately therefrom.

The calibration value may in particular depend on the liquid medium. Thus, different liquid media may have different calibration values.

In another embodiment, the method further comprises the step of selecting one liquid medium out of a list of liquid media, and providing a corresponding calibration ratio depending on the selected liquid medium. According to this embodiment, the liquid medium is known prior to the measurement and specified before a measurement. Said list of liquid media may be provided in a storage module of the calculating unit. The storage module may further comprise calibration ratios that correspond to a liquid medium. The liquid media and corresponding calibration ratios may be correlated to each other in a lookup table.

Another embodiment of the method comprises the step of determining a type and/or composition of a liquid medium, through which the ultrasonic pulse is transmitted, based on the calculated speed of sound signal.

This preferable embodiment of the method thus allows determining a) a type of the liquid medium and/or b) a specific composition of the liquid medium. In particular, in case of an urea solution this embodiment may be applied to measure an urea concentration independent on any transducer drifts or temperature influences as well as independent on disturbed conditions as for instance the presence of bubbles. This embodiment further provides a speed of sound in the liquid medium that it would be obtained without any disturbance.

In order to determine the type of liquid medium, another embodiment of the method compares the calculated speed of sound signal with a predetermined and/or stored list of the speed of sound signals. Thus, a list of possible liquid media is provided, e.g. in a lookup table, wherein additionally the speed of sound in those possible liquid media is related to those types of liquid media. By comparing the calculated speed of sound signal with a previously stored speed of sound signals, it is thus possible to assign the correct liquid medium.

According to the disclosure, the inventive apparatus may be configured to perform any embodiment of the inventive method described above. Thus, a corresponding apparatus may further comprise a reflector for reflecting the ultrasonic pulse at least twice for directing the first echo and the second echo towards the transducer, the apparatus may comprise corresponding calculation means provided additionally or within the calculator unit for measuring the time of flight, i.e. in particular for detecting a time span between generation of the ultrasonic pulse and detection of the first echo, comparator means for comparing measures with previously stored values and storage means for storing predetermined liquid media and corresponding calibration ratios and/or speed of sound signals.

Accordingly, the computer program product and the computer-readable storage medium may carry out the methods of any embodiment of the previously described method.

In the following, the invention is described by way of the accompanying figures, which describe exemplary embodiments of the present invention. Technical features of those exemplary embodiments may be arbitrarily combined with each other. Further, the exemplary embodiments do not limit a possible scope of protection, which is defined by the claims.

The figures show:
Fig. 1 an exemplary embodiment of the inventive apparatus;
Fig. 2 a flowchart of an exemplary embodiment of the inventive method;
Fig. 3 an exemplary measurement of an attenuation or the time indicating the influence of disturbances; and
Fig. 4 an exemplary comparison of a measured and corrected time of flight.

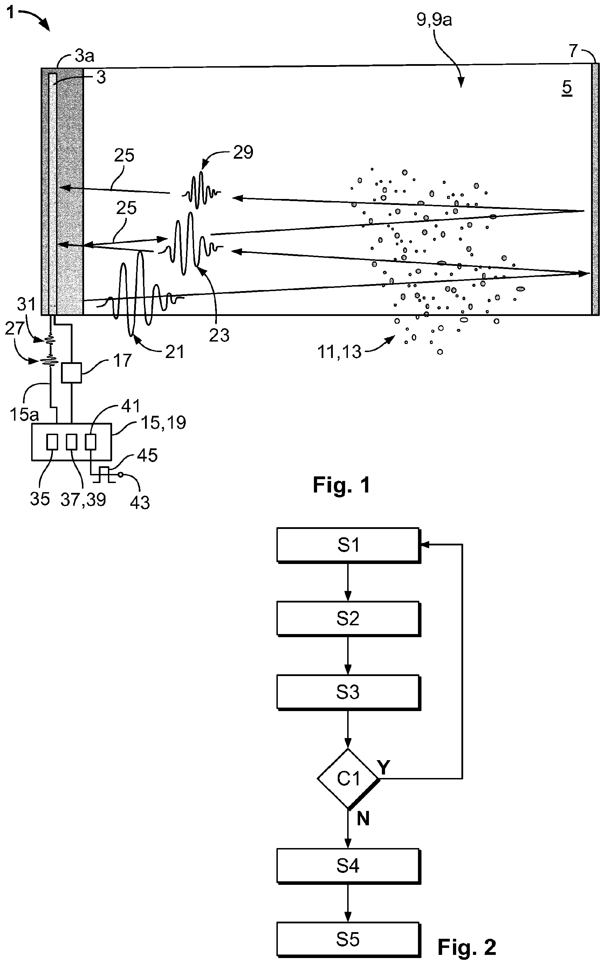

In fig. 1 an exemplary embodiment of the inventive apparatus 1 is shown. The apparatus comprises a pulse generator or transducer 3, which is provided in a transducer housing 3a. The transducer 3 is located adjacent to a sample volume 5, wherein opposite the transducer 3, a reflector 7 is provided.

In the sample volume 5 a liquid medium 9 in the form of an aqueous solution 9a is provided, wherein disturbances 11 in the form of bubbles 13 may occur in the aqueous solution 9a. the aqueous solution 9a may in particular be a urea solution 9b.

The apparatus 1 further comprises a calculator unit 15, which, in the embodiment shown, is connected to an electronic control unit 17 and to the transducer 3.

The calculator unit may also be a controller 19 that controls an output of the electronic control unit 17, which in turn drives transducer 3, i.e. provide the transducer 3 with an electronic signal, which is converted into an ultrasonic pulse 21 by the transducer 3.

In a more general embodiment, there may be provided a single and individual pulse generator and a single and individual receiver, which are not combined in one element. In the embodiment shown in Fig. 2 those two are combined.

The ultrasonic pulse 21 is transmitted through the aqueous solution 9a, is reflected at the reflector 7 and returns as a first echo 23 to the transducer 3. There, a portion 25 of the first echo 23 is absorbed in the transducer 3 and generates a first electrical or amplitude signal 27 that represents the first echo 23. This is indicated as a signal on a data line 15a of the calculator unit 15.

The first echo 23 is also reflected at the transducer and is transmitted towards the reflector 7 again. There, it is reflected and returns to the transducer 3 as a second echo 29. Again, a portion 25 of the second echo 29 is absorbed in the transducer 3 and generates a second electrical signal 31. Also the second electrical or amplitude signal 31 is schematically shown on the data line 15a. As the second echo 29 is attenuated, the second electrical signal 31 is smaller than the first electrical signal 27.

The time it takes for the ultrasonic pulse 21 from its generation to its arrival at the transducer 3 as the first echo 23 is a time of flight 55.

The calculator unit 15 may comprise a comparator unit 35, a storage module 37, comprising a lookup table 39 as well as an output unit 41 that comprises an output port 43 for providing a calculated speed of sound signal 45. The calculated speed of sound signal 45 is illustrated by a rectangular pulse on the output port 43.

As indicated in Fig. 1 the disturbances 11 in the form the bubbles 13 do influence the ultrasonic pulse 21 as well as the first echo 23 and the second echo 29, in particular the signal strength of the first 27 and the second electrical signal 31, as the ultrasonic pulse 17 as well as the first echo 23 and the second echo 29 are attenuated when being transmitted through the aqueous solution 9a and the bubbles 13 therein.

In Fig. 2 a flowchart showing one exemplary embodiment of the inventive method is shown. In step one S1 the ultrasonic pulse 21 is generated. In step two S2 the first echo 23 and the second echo 29 are received by the transducer 3 and converted into the corresponding first electrical signal 27 and the second electrical signal 31. Subsequently, in step three S3 an amplitude signal ratio between the amplitude signal of the first echo 23 and the amplitude signal of the second echo 29 is calculated.

Based on this amplitude signal ratio a condition one C1 is checked and verified. The amplitude signal ratio is compared with a predetermined amplitude ratio threshold and it is checked whether the amplitude ratio signal exceeds the amplitude ratio threshold.

If this is the case (branch indicated with 'Y') the entire method is restarted with step one S1. If the amplitude ratio threshold is not exceeded (branch indicated with ,N'), the method continues with step for S4, in which a compensation factor is determined, which is subsequently handed over to step five S5 in which the method determines a calculated speed of sound.

This flowchart only describes an exemplary embodiment of the inventive method. In different embodiments, additional steps may be provided, like e.g. outputting the determined calculated speed of sound and the like. Similarly, more conditions may be checked in different embodiments of the method.

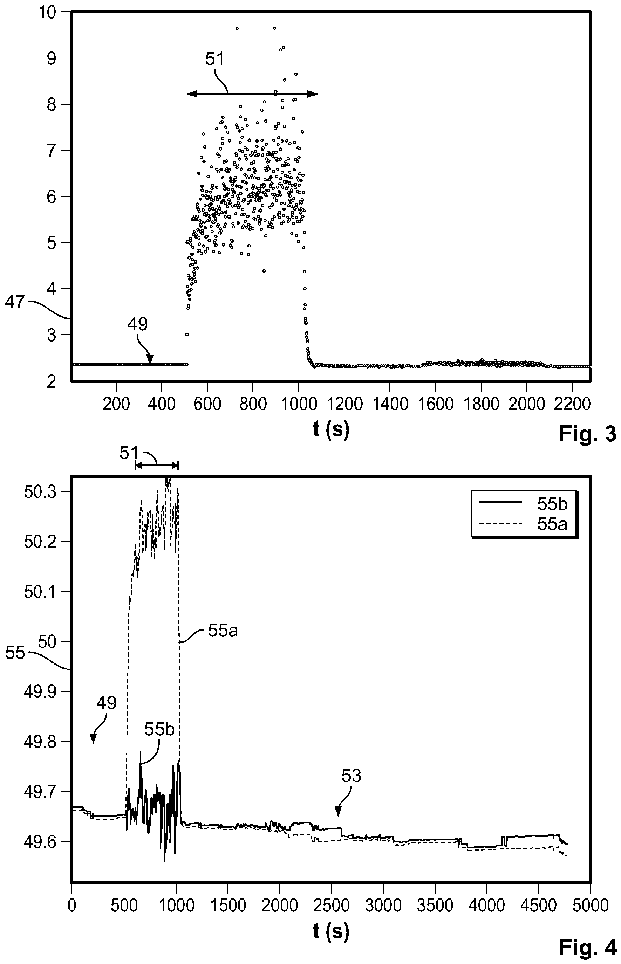

In Fig. 3 and Fig. 4 two exemplary measurements are shown to illustrate the effect of disturbances to an ultrasound measurement.

Fig. 3 shows an attenuation 47 depending on a time t. The attenuation 47 is functionally dependent on the amplitude signal ratio between the amplitude signal of the first echo and the amplitude signal of the second echo.

In a first region 49 no disturbances 11 are present in the liquid medium 9, wherein in a second region 51, disturbances 11 were present and clearly increased the attenuation 47. In a third region 53 again no disturbances 11 are present.

Fig. 4 shows the time of flight 55, having the unit of a time and corresponding to the time the ultrasonic pulse 21 takes for traveling through the sample volume 5 until it arrives again at the transducer 3, which is drawn over the time t.

The graph shows a measured time of flight 55a which is correlated with the attenuation 47 shown in Fig. 3. This correlation is applied to correct the time of flight, wherein after correction using the attenuation 47, a corrected time of flight 55b is obtained. The corrected time of flight 55b is drawn with a thicker solid line.

The increased time of flight 55 in the second region 51 may be explained by a higher speed of sound in bubbles 13 comprising a gaseous phase like air.

Fig. 4 clearly shows that even in the case of an increased attenuation from approximately 2.5 to a value of on average 6, the time of flight 55 may be accurately corrected to correspond to the time of flight 55 in the first region 49 and the third region 53, in which no disturbances 11 are present.

Consequently, the method allows determining a time of flight, and consequently a speed of sound in the liquid medium 9, which would be measured if no disturbances 11 were present. Properties of a pristine liquid medium 9 may thus also be acquired, respectively calculated if the liquid medium 9 already contains disturbances 11, e.g. in the form of bubbles 13.

### Reference Numerals

- 1: apparatus
- 3: transducer
- 3a: transducer housing
- 5: sample volume
- 7: reflector
- 9: liquid medium
- 9a: aqueous solution
- 9b: urea solution
- 11: disturbance
- 13: bubble
- 15: calculator unit
- 17: electronic control unit
- 19: controller
- 21: ultrasonic pulse
- 23: first echo
- 25: portion
- 27: first electrical signal
- 29: second echo
- 31: second electrical signal
- 35: comparator unit
- 37: storage module
- 39: lookup table
- 41: output unit
- 43: output port
- 45: calculated speed of sound signal
- 47: attenuation
- 49: first region
- 51: second region
- 53: third region
- 55: time-of-flight
- 55a: measured time-of-flight
- 55b: corrected time-of-flight

- t: time
- S1: step one
- S2: step two
- S3: step three
- S4: step four
- S5: step five
- C1: condition one

## Claims

1. Method for detecting a property of a liquid medium (9), in particular for detecting a property representative of disturbances (11) in the liquid medium (9), comprising the following steps:
- generating an ultrasonic pulse (21);
- receiving at least a first echo (23) and a second echo (29) of the ultrasonic pulse (21) transmitted through the liquid medium (9);
- generating an amplitude signal (27, 31) for each of the at least two echoes (23, 29);
- calculating an amplitude signal ratio between the amplitude signal (27) of the first echo (23) and the amplitude signal (31) of the second echo (29); and
- determining at least one property signal representing the property of the liquid medium (9).

2. Method according to claim 1, further comprising the step of determining a time of flight (55) for the first echo (23) for calculating a measured speed of sound and providing a measured speed of sound signal representing the measured speed of sound.

3. Method according to claim 2, further comprising the steps of
- determining a compensation factor based on the amplitude signal ratio and a predetermined calibration ratio;
- determining a calculated speed of sound in the liquid medium (9) at least based on the measured speed of sound and the compensation factor; and
- providing a calculated speed of sound signal (45) representing the calculated speed of sound.

4. Method according to claim 3, wherein the calculated speed of sound further depends on an empirically found variable.

5. Method according to any one of claims 1 to 4, further comprising the step of comparing the amplitude ratio signal with a predetermined amplitude ratio threshold, wherein if the amplitude ratio signal exceeds the amplitude ratio threshold, the method is re-initiated and/or an alert signal is provided.

6. Method according to any one of claims 3 to 5, wherein the calibration value depends on the liquid medium (9).

7. Method according to any one of claims 3 to 6, wherein the method further comprises the step of selecting one liquid medium (9) out of a list of liquid media (9), and providing a corresponding calibration ratio depending on the selected liquid medium (9).

8. Method according to any one of claims 3 to 7, further comprising the step of determining a type and/or composition of the liquid medium (9), through which the ultrasonic pulse (21) is transmitted, based on the calculated speed of sound signal (45).

9. Method according to claim 8, wherein the calculated speed of sound signal (45) is compared with a predetermined and/or stored list of speed of sound signals for determining the type of the liquid medium (9).

10. Apparatus (1) for detecting a property of a liquid medium (9), in particular for detecting a property representative of disturbances (11) in the liquid medium (9), the apparatus (1) comprising
- a pulse generator (3) configured to generate an ultrasonic pulse (21);
- a receiver configured to receive at least a first echo (23) and a second echo (29) of the ultrasonic pulse (21) transmitted through the liquid medium (9); and
- a calculator unit (15) configured to find and calculate an amplitude signal (27, 31) for each of the at least two echoes (23, 29), to calculate an amplitude signal ratio between the amplitude signal (27) of the first echo (23) and the amplitude signal (31) of the second echo (29) and to determine at least one property signal representing the property of the liquid medium (9).

11. Urea sensor system for determining at least one property of an urea solution (9b), in particular for detecting a property representative of disturbances (11) in the urea solution (9b), wherein the urea sensor system comprises an apparatus (1) according to claim 10.

12. Computer program product comprising instructions, which, when the program is executed by a computer, cause the computer to carry out the steps of the method of any one of claims 1 to 9.

13. Computer-readable storage medium comprising instructions which, when executed by a computer, cause the computer to carry out the steps of the method of any one of claims 1 to 9.
